# EUROPEAN PATENT SPECIFICATION

(11) **EP 3 331 492 B1**
(45) Date of publication and mention of the grant of the patent: **26.02.2025**
(21) Application number: 16716168.6
(22) Date of filing: 16.03.2016
(51) Int. Cl.: A61K 8/73, A61Q 19/00, A61K 31/737, A61P 17/04, A61P 17/16, A61P 43/00, A61P 17/00

(54) **COMPOSITIONS COMPRISING DERMATAN SULFATE AND CHONDROITIN SULFATE**
ZUSAMMENSETZUNGEN MIT DERMATANSULFAT UND CHONDROITINSULFAT
COMPOSITIONS COMPRENANT DU SULFATE DE DERMATANE ET DU SULFATE DE CHONDROÏTINE

(30) Priority: 06.08.2015 IT UB20152932; 05.02.2016 IT UB20160156
(43) Date of publication of application: 13.06.2018
(73) Proprietor: LABORATORI DERIVATI ORGANICI S.P.A., 20122 Milano (IT)
(72) Inventor: BENSI, Donata, 13100 Vercelli (IT); CAGLIO, Giovanni, 20122 Milano (IT)
(74) Representative: Serravalle, Marco
(86) International application number: PCT/EP2016/055652
(87) International publication number: WO 2017/021014

(56) References cited:
- EP-A1- 2 471 540
- WO-A1-2011/120535
- WO-A1-2014/013413
- WO-A1-97/47289
- WO-A2-01/85182
- FR-A1- 2 815 253
- JP-A- 2006 143 671
- JP-A- S6 422 812
- MIYAMOTO T ET AL: "Cosmetic material for skin conditioning - contg. one or more of hyaluronic acid, chondroitin and 6 sulphate and dermatan sulphate and mucin", WPI/THOMSON,, vol. 1987, no. 49, 30 October 1987 (1987-10-30), XP002512157

## Description

**.** The present invention is directed to a composition of glycosaminoglycans (GAGs) comprising dermatan sulfate and chondroitin sulfate, wherein the amount of dermatan sulfate is at least 20% and not more than 90 % by weight, and the amount of chondroitin sulfate is at least 10% and not more than 80 % by weight, based on the total amount of GAGs, wherein dermatan sulfate and chondroitin sulfate together are present in an amount that is at least 60 wt % of the total amount of GAGs, the amount of heparin is lower than 5 wt % and the amount of hyaluronic acid is lower than 20 wt %, characterized in that the weight ratio dermatan sulfate to chondroitin sulfate is comprised between 50/50 and 90/10.

**.** More particularly, the present invention is directed to a composition comprising from 0.1 to 15.0 wt %, preferably from 0.2 to 10.0 wt %, of a mixture of GAGs, which mixture comprises dermatan sulfate and chondroitin sulfate, wherein the amount of dermatan sulfate is at least 1% by weight based on the total amount of GAGs, and the amount of chondroitin sulfate is at least 1% by weight, based on the total amount of GAGs. The composition according to the invention are especially useful in the cosmetological field.

**.** The use of GAGs in the pharmaceutical field is well known in the art. Apart from the well-known use of low molecular weight heparin to prevent blood clots, other pharmaceutical uses of GAGs have been proposed. For example, US 2014/0343012 discloses the use of a combination of hyaluronic acid and another GAG, e.g. chondroitin sulfate, in the treatment of osteoarthritis.

**.** WO 2006/015171 discloses the use of various GAGs such as dermatan sulfate, chondroitin sulfate and LMW heparin for the treatment of sepsis.

**.** JP2006143671 relates to an oral skin aging preventive or ameliorating agent containing mucopolysaccharides, collagen and / or elastin or a partially degraded product thereof, and coenzyme Q10 as active ingredients, and oral skin containing these active ingredients.

**.** FR 2 815 253 discloses anti-age compositions comprising glycosaminoglycans such as hyaluronic acid, dermatan sulfate, chondroitin sulfate, heparan sulfate, heparin, keratan sulfate. All examples make use of hyaluronic acid.

**.** It has been surprisingly found that a skin product containing a mixture comprising dermatan sulfate and chondroitin sulfate is very effective as anti-age composition, to reduce wrinkles and maintain the skin hydrated.

**.** The amount of dermatan sulfate is at 20 wt %, or at least 40 wt %, or at least 50 wt %, or at least 60 wt %, based on the total amount of GAGs.

**.** The amount of chondroitin sulfate is at least 10 wt %, or at least 20 wt % based on the total amount of GAGs.

**.** Concerning the weight ratio dermatan sulfate/chondroitin sulfate, it is comprised from 50/50 to 90/10, or 85/15, or 80/20, or 75/25. Any range derived from the combination of the above defined limits of the ratio are disclosed in the present description. For example, the range can be from 50/50 to 85/15.

**.** Although the presence of both dermatan sulfate and chondroitin sulfate is essential, it is not excluded that other GAGs might be present.

**.** GAGs (or mucoplysaccharides) are long unbranched polysaccharides consisting of a repeating disaccharide unit. The repeating unit (except for keratan) consists of an amino sugar (N-acetylglucosamine or N-acetylgalactosamine) along with a uronic sugar (glucuronic acid or iduronic acid) or galactose.

**.** Non-limiting examples of GAGs are: dermatan sulfate, chondroitin sulfate, heparan sulfate, heparin, depolymerized heparin, e.g. low molecular weight heparin and very low molecular weight heparin, chemically modified heparin, e.g. supersulfated heparin, hyaluronic acid, keratan sulfate.

**.** It is preferred that dermatan sulfate and chondroitin sulfate together are present in an amount that is at least 60 wt %, preferably at least 70 wt % and most preferably at least 80 wt %.

**.** The GAGs composition contains an amount of hyaluronic acid lower than 20 wt %, or lower than 10 wt %, or lower than 5 wt %. More preferably, the GAGs composition is substantially free of hyaluronic acid. The amount of heparin is lower than 5 wt %. In a particularly preferred embodiment, the composition is substantially free of heparin. Preferably, the amount of keratan sulfate is also limited and is preferably lower than 20 wt %, or lower than 10 wt %, or lower than 5 wt %. More preferably, the GAGs composition is substantially free of keratan sulfate.

**.** The composition of the invention can be suitable for use in the treatment of aging skin or skin pathologies (e.g. atopic skin, dry/very dry skin, itching skin, etc.). For example, the composition can be in the form of an aqueous solution or of a cream.

**.** Typically, a cosmetological composition is formulated as a solution or a cream. A solution is an aqueous monophasic system, which preferably comprises from 30 to 98 wt % water, more preferably from 85 to 97 wt %; aqueous solutions can be also in the form of gel, where the structure is generally achieved thanks to a stabilization due to specific rheological ingredients, i.e. gel formers, preferably in an amount comprised between 0.1 and 10 wt %, more preferably between 0.2 and 5 wt %. The composition can further comprise other additives such as humectants, anti-oxidants, preservatives, stabilizers, and other compounds which are commonly used in cosmetic gels.

**.** If the composition is in the form of a cream, either an oil and/or silicon in water or a water in oil and/or silicon emulsion, water is normally present in a lower amount preferably comprised between 0 and 75 wt %, more preferably from 30 to 70 wt %, more preferably from 60 to 70 wt %; in this case, the composition also includes at least one oil and/or silicon or fat selected from natural and synthetic oils/fats or mixtures thereof in an amount preferably comprised between 5 and 50 wt %, more preferably from 8 to 30 wt %, most preferably from 10 to 20 wt %; and one or more emulsifiers selected from ionic and non-ionic emulsifiers in an amount preferably comprised between 1 and 20 wt %, more preferably between 2 and 15 wt %, most preferably between 4 and 14 wt %. Thus, a preferred composition according to the invention in the form of a cream will comprise at least one synthetic or natural oil or fat, and optionally water, emulsifiers, preservatives, anti-oxidants, humectants, and other compounds which are usually used in cosmetic creams.

**.** The content of the GAGs composition according to the invention in a cosmetological composition is at least 0.1 wt %, or at least 0.2 wt %, or at least 0.3 wt %, or at least 0.5 wt %, or at least 0.8 wt %. It is also preferred that the glycosaminoglycans composition is present in amounts not higher than 15.0 wt %, or not higher than 10.0 wt %, or not higher than 5.0 wt %, or not higher than 3.0 wt %, or not higher than 2.0 wt %, or not higher than 1.5 wt %, or not higher than 1.3 wt %. In a most preferred embodiment, the composition is present in an amount of about 1 wt %, e.g. from 0.9 wt % to 1.1 wt %. Independently from other GAGs or non-GAGs components present in the cosmetological composition, it is preferred that the amount of dermatan sulfate and chondroitin sulfate together represents at least 0.1 wt %, preferably at least 0.2 wt %, more preferably at least 0.4 wt %, even more preferably at least 0.5 wt % of the total cosmetological composition.

**.** On the other hand, the amount of dermatan sulfate and chondroitin sulfate together is preferably lower than 10 wt %, more preferably lower than 5 wt %, even more preferably lower than 3 wt % based on the total weight of the cosmetological composition.

**.** Examples of a gel composition and of a cream are the following:
**.** Gel:

| | |
|---|---|
| Water: | 96.45% |
| Sepiplus S (stabilizer) | 3.0% |
| GAGs composition | 0.5% |
| Kathon (preservative) | 0.05% |

Cream

| | |
|---|---|
| Water phase: | |
| Water | 66.65% |
| Glycerin (humectant) | 4.0% |
| Xanthan gum (stabilizer) | 0.2% |
| EDTA bisodic salt (scavenger) | 0.1% |
| Sodium Benzoate (Preservative) | 0.5% |
| Citric acid (pH corrector) | 0.25% |
| GAGs composition | 0.5% |
| Emulsifier (Sodium Olivoyl Glutamate, Cetearyl Alcohol, Glyceryl Stearate) | 5.0% |
| Stabilizer (Polyacrylamide, aqua, C13-14 Isoparaffin, Laureth-7, Sepigel 305) | 3.0% |
| Oil phase: | |
| Hydrogenated Olive Oil, Olive Oil, Olive Oil Unsaponifiable | 1.0% |
| Dimethicone | 1.0% |
| C12-15 Alkyl Benzoate | 10.0% |
| Glyceryl stearate | 4.0% |
| Cetearyl Alcohol | 2.5% |
| Parfum | 0.5% |
| Phenoxyethanol | 0.8% |

**.** It has been found that the use of the GAGs composition according to the invention in skin products results in an improvement of the skin matrix, which improvement produces an anti-age, anti-wrinkle and moisturizing effect.

**.** The skin matrix is responsible for structural integrity, mechanical resilience, stability and many other properties of the skin. The degradation of the skin matrix plays an important role in the development of wrinkles and other signs of skin ageing. The best known components of the skin matrix are structural proteins (collagen and elastin), which are vital to skin health and youthfulness. Structural proteins are necessary but insufficient for a healthy skin matrix. Hyaluronic acid, the principal skin matrix filler, provides mechanical cushioning, holds moisture and is responsible for long lasting moisturizing properties.

**.** Decorin is the main proteoglycan present in the skin; it is a small proteoglycan with a core protein, which is horseshoe-shaped and whose inner concavity accommodates and may provide a binding site for type I collagen. The amount of decorin in the matrix skin have a significant effect on skin elasticity. In fact, a specific ratio decorin to collagen is necessary for optimal fiber formation.

**.** The composition according to the invention is effective in increasing the long term hydration of the skin, and reducing depth of wrinkles.

**.** Without being bound to any theory, it is possible that the moisturizing, anti-age and anti-wrinkle effect of the composition according to the invention is related to its effect in increasing decorin, collagen I and hyaluronic acid synthetase gene expression.

### Experimental part

**.** Concentration of dermatan sulfate and chondroitin sulfate in the GAGs composition were measured by electrophoresis according to the following method.

**.** A solution of the GAGs composition at a concentration of about 0.7% in purified water was prepared. The cryostat was switch on and the basin for electrophoresis was cooled down. The lateral tanks of the basin were filled with 1 M Barium acetate buffer solution and the central one with n-Decane. The two liquids are not miscible: decane, being lighter, remained on the surface and was used to keep constant the buffer temperature.

**.** A plexiglass tank was filled with purified water and another one with a solution of 0.1 M Barium acetate. One side of the plate was immersed in purified water to a depth of about 1 cm, dryed with paper towels, and the remaining part imbued with the 0.1 M Barium acetate solution, leaving few mm of dry plate between water and barium acetate. It was dried with paper towels and, using the applicator, the samples were sown and any standard in the part of plate soaked in water.

**.** The plate was placed in the electrophoresis cell, on the bridge, with the porous part facing downwards and the side on which samples were sown, facing the cathode (negative pole). The 1^{st} electrophoresis run was performed for 2 minutes at 200 volts. The samples migrated on the line between the two solutions (water and Barium acetate); the plate was removed, dried and immersed for exactly 2 minutes in the 3% Ethanol solution. In this way the migration of the Slow Moving band was stopped. The plate was dried, placed back in the cell and the 2^{nd} run performed for 15 minutes at 200 volts. At the end of the run, the plate was removed, dried and placed in the 20% Ethanol solution for exactly 2 minutes. In this way the Fast Moving (dermatan sulfate) bands were stopped.

**.** The plate was dried and placed in the cell for the 3^{rd} electrophoresis run, at 200 volts for 25 minutes; in this way the chondroitin sulfate band migrates. Finally, the plate was dried and transferred in the Staining solution for 10 minutes, then in the decolorizing solution till complete discoloration was obtained, changing the solution occasionally.

**.** The reading of the plate to the densitometer was performed in the following conditions:
- Wavelength: 600 mm
- Photo mode: reflection

The instrument produced a report where the migration bands are shown as a chromatogram. For each peak are displayed the values of electrophoretic mobility (Rf), area and area% (area of each peak x 100/sum of areas of all peaks).

**.** Detection of hyaluronic acid can be performed by HPLC according to conventional techniques. Heparin and heparan sulfate are also detected by electrophoretic techniques.

### In vitro experiments

**.** The in vitro experiments were conducted on a solution comprising 1% of a GAGs composition according to the invention. The GAGs composition consisted of 75 wt % dermatan sulfate and 25 wt % chondroitin sulfate. The experiments were conducted on 3D tissues.

**.** Between in vitro alternatives, 3D tissues have the strong advantage of representing the biological model closest to humans: they have a multilayered structure and a tissue functionality as the in vivo tissues; furthermore, the products to be tested can be applied on the tissue surface at the same concentration and mode as in vivo.

**.** The treatment of human skin with topically applied products leads to a genomic response which has a dynamic pathway and represents the first cellular signal at transcriptional level responsible for a cascade of events. 3D living human tissues are relevant test systems to assess efficacy taking into account either the direct genomic response and also the result of cellular communication and crosstalk via soluble mediators and specific biomarker expression.

**.** The concept of "cosmetogenomics" was applied to set-up experimental models for predicting the efficacy of ingredients and finished products. A homeostasis model was specifically developed for cosmetic product testing on "Full-thickness skin model" (FT-skin) reproducing dermal and epidermal compartments: this model has the specificity of allowing the study of dermal extracellular matrix modification after short term (24h/ 48H) and long term (up to 2 weeks) after product application. The relative efficacy of the product is quantified compared to control untreated tissues.

**.** The efficacy of the active 1 wt % solution was evaluated in vitro on FT skin model on skin homeostasis conditions after a treatment of 24h and 48h.

**.** The Phenion^{®} Full Thickness Skin Model is produced by Henkel (Düsseldorf, Germany, diameter 1.3 cm). In this model, epidermal keratinocytes and dermal fibroblasts (derived from biopsy material from healthy donors) form a multilayered skin equivalent that resembles human skin under culture conditions. Fibroblasts are grown in a specialized stable matrix that does not contract under fibroblast traction forces. After the development of this dermal equivalent, keratinocytes are overlaid and within a few days they develop an epidermis with clearly recognizable layers. Both the epidermis and dermis form a physiologically functional unit, and like human skin, the epidermis produces various markers of differentiation (cytokeratin 10, filaggrin, transglutaminase, and involucrin). The epidermal-dermal junction is characterized by basal membrane proteins (laminin and collagen IV). In the dermal compartment, de novo synthesis of elastin and fibronectin has been demonstrated. The proliferative cells of the basal layer is identified by Ki-67 staining. The model is fully developed after a cultivation period of 5 weeks.

. Treatment: 100 µ L of the 1% solution and of saline solution as negative control were applied on duplicate cultures on homeostasis conditions (no stress applied) on the epidermis surface for 24h and 48h. The product was re-applied after 24h. The following biomarkers have been analyzed by real-time reverse transcription polymerase chain reaction (qRT-PCR): Decorin, Collagen I and Hyluronate Synthetase 1. Protein level was analyzed by complementary immunofluorescence qualitative analysis. Decorin and Collagen I protein expression have been quantified by Western Blotanalysis.

### RNA extraction, cDNA Retrotranscription and REAL TIME PCR

**.** For the three steps, ready to use reagents were used. The RNAqueous method is a rapid, phenol free, filter based RNA isolation system used to extract the total RNA from cellular samples. The High Capacity cDNA Reverse Transcription kit was used to synthetize cDNA from RNA. The instrument Applied Biosystems 7500 Fast Real Time PCR with fluorescent-based PCR chemistry, the TaqMan assay, was used to study gene expression of significant biomarkers. Gene expression is the process by which the inheritable information in a gene, such as the DNA sequence, is made into a functional gene product, such as protein or RNA. Relative quantification determines the change in the expression of a nucleic acid sequence in a test sample relative to the same sequence in a calibrator sample. GAPDH was used as an endogenous control gene to normalize input amounts. Each replicate was assessed in triplicate. At the 2X TaqMan Fast Universal PCR Master Mix was added Taqman gene expression assay and cDNA (25 ng) for a total volume of 25 µL. The Thermal condition steps in the ABI PRISM 7500 Fast are: 95°C 20 sec; 40 cycles (95°C 3 sec +60°C 30 sec).

### WESTERN BLOT ANALYSIS

### Principle of the method

**.** Western blotting is a well-established and widely used technique for the detection and analysis of proteins. The method is based on the building of an antibody: protein complex via specific binding of antibodies to proteins immobilized on a membrane and detecting the bound antibody with one of several detection methods. A protein sample is subjected to polyacrylamide gel electrophoresis. The gel is then placed over a sheet of nitrocellulose and the protein in the gel is electrophoretically transferred to the nitrocellulose. The nitrocellulose is then soaked in blocking buffer to "block" the non- specific binding of proteins. The nitrocellulose is then incubated with the specific antibody for the protein of interest. The nitrocellulose is then incubated with a second antibody, which is specific for the first antibody. The secondary antibody will typically have a covalently attached enzyme which, when provided with a chromogenic substrate, will cause a color reaction.

### Procedure

**.** FT-SKIN has been placed in BIO-PLEX CELL LYSIS buffer (BIORAD) with freshly added protease inhibitor mixture, homogenized, and stored on ice for 30 min, then centrifuged at 16,000 x g for 10 minutes at 4°C. Protein determination have been performed with RC DC Protein Assay (BIORAD). After adding an equal volume of 2X Laemmli Sample Buffer to the lysate, each sample is boiled in sample buffer at 100°C for 5 minutes and ready (50 µg of total protein) to be separated on Tris·Glycine Extended (TGX) Stain-free precast gels 7,5% (Bio-Rad) and transferred to PVDF membrane (BIORAD). The membrane has been blocked for 1 hour at room temperature using 5% blocking solution (BSA) in TBST 0,05%. The membranes have been incubated with rabbit polyclonal antibody anti DCN (NBP-84970, NovusBio) and anti COL1 (PA5-29569, Thermo Scientific), for 1h incubation at room temperature. The membrane has been rinsed in three washes of TBST, 5 minutes each.
Bounded antibodies have been detected with the recommended dilution of labeled goat anti-rabbit IgG secondary antibody (656120, Invitrogen) in in TBST at room temperature for 1 hour. After three washes in TBST, the membrane is ready for signal development with CLARITY WESTERN ECL SUBSTRATE (BIORAD). The chemiluminescence bands of DCN and COL1 have been visualized by using Chemidoc XRS system (BIORAD) and quantified by using ImageLab software (100/50KDa and 250 kDa respectively).

**.** After 48 hours from the treatment, the increase in decorin gene expression is 46 %, the increase in collagen I gene expression is 61 % and the increase in hyaluronic acid synthetase gene expression is 559 %.

### In vivo experiments

. The test was performed on 25 volunteers for 4 weeks of treatment. The volunteers were treated with an oil in water emulsion cream as above defined.

**.** The GAGs composition consisted of 75 wt % dermatan sulfate and 25 wt % chondroitin sulfate. As a reference, the identical cream without GAGs was used (base emulsion).

**.** Two measures were performed on the 25 volunteers: wrinkle depth and Hydration level. Wrinkle depth was evaluated with Dermatop Blue^{™}. Hydration level was measured by corneometry after 3 h from the first application and after 8 days of application twice a day.

### Anti-wrinkle effect

**.** The volunteers using the cream according to the invention showed an average wrinkles depth reduction after 4 weeks of 23.1% versus a reduction of 16.4% of the volunteers using the base emulsion.

### Moisturizing effect

**.** After 3 h from the first application, the base emulsion appeared more effective than the cream according to the invention (base emulsion +39.9% against inventive cream +28.7%); however, after 8 days the picture was completely changed (base emulsion +33.5% versus +52.3% of the inventive cream).

**.** These data demonstrate that in vitro data are confirmed by in vivo tests. The use of a GAGs composition comprising dermatan sulfate and chondroitin sulfate produces a moisturizing effect and a statistically relevant reduction of wrinkle depth.

## Claims

1. A composition of glycosaminoglycans comprising dermatan sulfate and chondroitin sulfate, wherein the amount of dermatan sulfate is at least 20% and not more than 90 % by weight, and the amount of chondroitin sulfate is at least 10% and not more than 80 % by weight, based on the total amount of glycosaminoglycans, wherein dermatan sulfate and chondroitin sulfate together are present in an amount that is at least 60 wt % of the total amount of GAGs, the amount of heparin is lower than 5 wt % and the amount of hyaluronic acid is lower than 20 wt %, **characterized in that** the weight ratio dermatan sulfate to chondroitin sulfate is comprised between 50/50 and 90/10.

2. The composition according to claim 1, further comprising heparan sulfate.

3. The composition according to claim 1, wherein dermatan sulfate and chondroitin sulfate together are present in an amount that is at least 80 wt % of the total amount of GAGs.

## Patentansprüche

1. Zusammensetzung von Glykosaminoglykanen, umfassend Dermatansulfat und Chondroitinsulfat, wobei die Menge an Dermatansulfat mindestens 20 Gew.-% und nicht mehr als 90 Gew.-% beträgt und die Menge an Chondroitinsulfat mindestens 10 Gew.-% und nicht mehr als 80 Gew.-% beträgt, basierend auf der Gesamtmenge an Glykosaminoglykanen, wobei Dermatansulfat und Chondroitinsulfat zusammen in einer Menge vorliegen, die mindestens 60 Gew.-% der Gesamtmenge an GAGs beträgt, die Menge an Heparin weniger als 5 Gew.-% und die Menge an Hyaluronsäure weniger als 20 Gew.-% beträgt, **dadurch gekennzeichnet, dass** das Gewichtsverhältnis von Dermatansulfat zu Chondroitinsulfat zwischen 50/50 und 90/10 liegt.

2. Zusammensetzung nach Anspruch 1, ferner umfassend Heparansulfat.

3. Zusammensetzung nach Anspruch 1, wobei Dermatansulfat und Chondroitinsulfat zusammen in einer Menge vorliegen, die mindestens 80 Gew.-% der Gesamtmenge an GAGs beträgt.

## Revendications

1. Composition de glycosaminoglycanes comprenant du dermatane sulfate et du sulfate de chondroïtine, dans laquelle la quantité de dermatane sulfate est d'au moins 20 % et pas plus de 90 % en poids, et la quantité de sulfate de chondroïtine est d'au moins 10 % et pas plus de 80 % en poids, sur la base de la quantité totale de glycosaminoglycanes, dans laquelle le dermatane sulfate et le sulfate de chondroïtine sont présents ensemble en une quantité qui est d'au moins 60 % en poids de la quantité totale de GAGs, la quantité d'héparine est inférieure à 5 % en poids et la quantité d'acide hyaluronique est inférieure à 20 % en poids, **caractérisée en ce que** le rapport en poids du dermatane sulfate au sulfate de chondroïtine est compris entre 50/50 et 90/10.

2. Composition selon la revendication 1, comprenant en outre de l'héparane sulfate.

3. Composition selon la revendication 1, dans laquelle le dermatane sulfate et le sulfate de chondroïtine sont présents ensemble en une quantité qui est d'au moins 80 % en poids de la quantité totale de GAGs.
